# EUROPEAN PATENT SPECIFICATION

(11) **EP 1 009 442 B1**
(45) Date of publication and mention of the grant of the patent: **16.04.2003**
(21) Application number: 98940480.1
(22) Date of filing: 07.09.1998
(51) Int. Cl.: A61K 48/00, C12N 5/10, A61P 35/00

(54) **IMPROVEMENTS IN OR RELATING TO REGULATION OF T CELL ACTIVATION**
VERBESSERUNGEN DER ODER IN BEZUG AUF DIE REGULIERUNG DER T-ZELLAKTIVATION
AMELIORATIONS DE OU RELATIVES A LA REGULATION DE L'ACTIVATION DES CELLULES T

(30) Priority: 06.09.1997 GB 9718872; 07.11.1997 GB 9723448; 02.03.1998 US 76448 P
(43) Date of publication of application: 21.06.2000
(73) Proprietor: Mayo Foundation for Medical Education and Research, Rochester, MN 55906 (US)
(72) Inventor: ALVAREZ-VALLINA, Luis Hospital Universitario, E-28035 Madrid (ES); RUSSELL, Stephen, James, Rochester, MN 55902 (US); HAWKINS, Robert, Prestbury, Cheshire SK10 4YG (GB); AGHA-MOHAMMADI, Siamak, Pittsburgh, PA 15213 (US)
(74) Representative: Lipscombe, Martin John
(86) International application number: GB9802689
(87) International publication number: WO99012573

(56) References cited:
- WO-A-96/40892
- ESHHAR ET AL: "SPECIFIC ACTIVATION AND TARGETING OF CYTOTOXIC LYMPHOCYTES THROUGH CHIMERIC SINGLE CHAINS CONSISTING OF ANTIBODY-BINDING DOMAINS AND THE GAMMA OR ZETA SUBUNITS OF THE IMMUNOGLOBULIN AND T-CELL RECEPTORS" PROCEEDINGS OF THE NATIONAL ACADEMY OF SCIENCES, USA, vol. 90, 1993, pages 720-724, XP002088319 cited in the application
- WANPING JIANG ET AL: "THE RECEPTOR DEC-205 EXPRESSED BY DENDRITIC CELLS AND THYMIC EPITHELIAL CELLS IS INVOLVED IN ANTIGEN PROCESSING" NATURE, vol. 375, no. 6527, 11 May 1995, pages 151-155, XP000571400 cited in the application
- MILLER ET AL: "PROGRESS IN TRANSCRIPTIONALLY TARGETED AND REGULATABLE VECTORS FOR GENETIC THERAPY" HUMAN GENE THERAPY, vol. 8, 1 May 1997, pages 803-815, XP002088320 cited in the application
- VALITUTTI ET AL: "SERIAL TRIGGERING OF MANY T-CELL RECEPTORS BY A FEW PEPTIDE-MHC COMPLEXES" NATURE, vol. 375, 1995, pages 148-151, XP002088368 cited in the application
- ALVAREZ-VALLINA ET AL: "PHARMACOLOGICAL CONTROL OF ANTIGEN RESPONSIVENESS IN GENETICALLY MODIFIED T LYMPHOCYTES" THE JOURNAL OF IMMUNOLOGY, vol. 159, 1997, pages 5889-5895, XP002088321 cited in the application

### SEQUENCE LISTING

(1) GENERAL INFORMATION:
   (i) APPLICANT:
      (A) NAME: Medical Research Council
      (B) STREET: 20 Park Crescent
      (C) CITY: London
      (E) COUNTRY: United Kingdom
      (F) POSTAL CODE (ZIP): W1N 4AL
      (G) TELEPHONE: (0171) 636 5422
      (H) TELEFAX: (0171) 323 1331
   (ii) TITLE OF INVENTION: Improvements in or Relating to T cell Activation
   (iii) NUMBER OF SEQUENCES: 2
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0. Version #1.30 (EPO)
(2) INFORMATION FOR SEQ ID NO: 1:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO: 1:
   CATCGATCGA ACTGATATCA GCAGATCTCA GAAGCTTAAT 40
(2) INFORMATION FOR SEQ ID NO: 2:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (xi) SEQUENCE DESCRIPTION: SEQ ID NO: 2:
   ATTAAGCTTC TGAGATCTGC TGATATCAGT TCGATCGATG ACGT 44

## Description

### Field of the Invention

This invention relates, *inter alia,* to a method of altering the sensitivity of a leukocyte to a target antigen.

### Background of the Invention

Unlike antibody molecules, T cells can migrate actively and efficiently through microvascular walls, allowing them to penetrate the core of a solid tumor before they exert their cytolytic function. *Ex vivo* expansion and re-infusion of autologous tumor-reactive T cells is being explored as an experimental approach to cancer therapy. However, circulating T cells from peripheral blood lack specificity for tumor antigens (1) and it is often impractical or impossible to obtain sufficient numbers of tumor infiltrating lymphocytes (2). To overcome these problems new approaches have been developed whereby antibody specificity can be combined with the efficient trafficking properties and effector functions of T cells. Several reports have demonstrated the feasibility of transfecting cultured T cells with genes encoding chimeric receptors in which single-chain antibody domains (scFv) are linked to different signalling portions of the TCR/CD3/ζ complex as a means to target T cells towards native antigens (3-6). However it is apparent that the long-term clinical success of this "T-body" approach could depend on the development of solutions to a number of problems.

Perhaps the most significant concern is that since few "cancer antigens" are truly tumor-specific (7), successful therapy with tumor-reactive T-bodies could be associated with significant collateral damage to normal tissues expressing low levels of the targeted antigen. It will therefore be desirable to develop strategies by which T cells can be rendered temporarily or permanently anergic to their target antigen, or differentially sensitive to different surface densities of the antigen on target cell membranes.

Although different strategies have been developed for regulating transgene expression in eukaryotic cells (8), the tetracycline-regulatable system (TRS) avoids the problems related to many of these systems by offering substantial regulation of transgene expression in response to concentrations of tetracycline that cause little or no toxicity in mammalian cells (9, 10).

Miller & Whelan (1997 Hum. Gene Therapy **8,** 803-815) have recently reviewed progress towards the development of regulatable vectors for gene therapy. Among the vectors described are those using the TRS.

In the TRS originally described by Gossen & Bujard (1992 Proc. Natl. Acad. Sci. USA **89**, 5547-5551), the tetracycline repressor protein was fused to the Herpes Simplex Virus (HSV) VP16-activating domain, to create a chimeric tetracycline-repressible transactivating (tTA) polypeptide, which binds to DNA comprising the tet operator sequence, causing transcriptional activation of coding sequences downstream of the operator. The presence of tetracycline or analogues thereof (such as doxycycline, anhydrotetracycline, minocycline and oxytetracycline) inhibits this transcriptional activation, as these compounds bind to the tTA polypeptide, altering its conformation and prevent its binding to the tet operator sequence.

Variants of the original TRS have now been described (WO 96/01313) in which a mutant form of the tet repressor protein binds to DNA in the presence, but not in the absence, of tetracycline or its analogues. Thus, in these systems, expression of a tet operator-linked gene is positively regulated in the presence of tetracycline or its analogues.

However, there are a number of difficulties and/or uncertainties relating to the use of the TRS. For example, Cooke *et al*, (1997 J. Gen. Virol. **78,** 381-392) found that the TRS could not be used to regulate expression of the *nef* gene in the human T cell line Jurkat E6-1. Further, the HSV VP16 domain is associated with a toxic "squelching" effect, such that high levels of regulation have not previously been obtainable. A recent review (Miller & Whelan 1997 Hum. Gene Ther. 8, 803-815) has stated that the TRS "may not be applicable to all cell lines" (citing the work of Ackland-Berglund & Leib 1995 BioTechniques *18*, 196-200; and Howe *et al.*, 1995 J. Biol. Chem. *270,* 14168-14174). WO 96/40892 discloses the use of a tetracycline repressor/operator/inducer system to regulate gene expression in prokaryotic cells or to modulate the expression of a therapeutic gene in gene therapy applications. The document does not explicitly mention that transformed cells for use in gene therapy applications should be leukocytes and moreover does not teach or in any way suggest that the tet expression system could be used to regulate the reactivity of a leukocyte so as to distinguish between target and non-target cells.

### Summary of the Invention

The present specification discloses a method of making a leukocyte differentially reactive to different densities of leukocyte-stimulating molecules present on the surface of a cell, wherein the leukocyte is activated by an interaction between the leukocyte-stimulating molecule on the cell and a leukocyte-activating molecule present on the surface of the leukocyte, the method comprising: transforming the leukocyte with a nucleic acid sequence which directs the expression of the leukocyte-activating molecule in a manner sensitive to the concentration of an exogenous agent; and altering the concentration of exogenous agent to which the leukocyte is exposed. Typically the exogenous agent is a "regulatory drug", as explained below, and the nucleic acid sequence directing the expression of the leukocyte-activating molecule is operably linked to a drug-regulatable promoter.

The exogenous agent may be any agent which affects the expression of the leukocyte-activating molecule, preferably in a selective, specific manner. Preferably the exogenous agent is one which can be administered to a human patient in a pharmacological manner: that is, the agent exerts an appropriate effect on the expression of the leukocyte-activating molecule at a concentration lower than that which causes any significant toxic effect on the patient. For example, the leukocyte-activating molecule is preferably expressed in a regulatable manner (e.g. using a tet O sequence and a tet-sensitive transactivator), substantially as described below. In this embodiment, the exogenous agent may be tetracycline or an analogue thereof.

A convenient way of rendering the expression of the leukocyte-activating molecule in a manner sensitive to the concentration of an exogenous agent, is to select as the exogenous agent a suitable "regulatory drug", and to place the sequence encoding the leukocyte-activating molecule under the control of a drug-regulatable promoter, whose activity is controlled directly or indirectly, by the concentration of drug. The term "drug-regulatable promoter" is intended to refer to portions of nucleic acid (e.g. enhancers, or more preferably, promoters) which regulate the expression of coding regions of nucleic acid, in response to the presence of a substance (the "regulatory drug") exogenous to the cell.

The regulatory drug is such that it causes modification of the activity of the drug-regulatable promoter at a concentration which is non-lethal to the cell in which the nucleic acid is present. Preferably the action of the regulatory drug is substantially specific for the drug-regulatable promoter such that, if it is necessary to administer the drug to the patient it will not cause widespread effects on other tissues or cell types. Such specificity is most readily conferred by use of a synthetic regulatory drug and a corresponding drug-regulatable promoter which is not normally present in the subject. The drug-regulatable promoter may be directly drug-regulatable, or more typically, indirectly drug-regulatable. An example of a system comprising an indirectly drug-regulatable promoter is a system in which a drug-regulatable operator exerts a regulatory effect on a promoter, which effect depends on the concentration of regulatory drug.

A number of suitable drug-regulatable promoters and corresponding regulatory drugs are known (see for example, Miller & Whelan, Hum. Gene Ther. 8, 803-815), and include promoters regulated by glucocorticoid steroids, sex hormone steroids, lipopolysaccharide (LPS) and isopropylthiogalactoside (IPTG). Suitable concentrations of the corresponding drugs will be apparent to those skilled in the art. As a general guide, when culturing cells *in vitro* in the presence of steroids a preferred concentration for the steroid drug is 1nM to 1mM. When the steroid drug is administered *in vivo* to a human or animal subject, the dosage is desirably adjusted to achieve a serum concentration beween about 0.05 and 10.0 µg/Kg. When culturing cells in the presence of IPTG, the dosage is preferably in the range of 1µM to 100mM, more preferably in the range 100µM to 1mM. When culturing cells in the presence of LPS the dosage is conveniently in the range 0.1nM to 1mM, more desirably in the range 100µM to 1mM.

Another system, particularly useful in applying the present invention, is the tetracycline-regulatable system (TRS), which has been extensively studied and several variants thereof have been developed, making the TRS extremely adaptable. For example, in the absence of tetracycline the wild-type bacterial tet repressor protein causes negative regulation of bacterial tetracycline-resistance genes: tetracycline binds to the repressor protein and prevents it from binding to the tet operator DNA sequence, thus allowing expression of the resistance genes. Conversely, a chimeric polypeptide comprising part of the tet repressor and the HSV VP16 transactivating domain causes positive regulation (transcriptional activation) of coding sequences. Other transactivating domains are known to those skilled in the art (e.g. amino acid residues 753-881 of GAL4; amino acid residues 399-499 of CTF/NF1; and those from ITF1 or ITF2), and these may conceivably be used to form a chimeric tetracycline-sensitive polypeptide.

In addition to the chimeric polypeptides described in the prior art (e.g. Gossen & Bujard 1992, cited above) which comprise a portion of the wild type tet repressor protein, other polypeptides are known (disclosed in WO 96/01313) which comprise a mutated form of the tet repressor protein, which binds to the tet operator sequence in the presence, but not in the absence, of tetracycline.

Accordingly the invention is such that in some embodiments the presence of tetracycline will serve to inhibit expression of a tetracycline operator (tet O) - linked coding sequence (i.e. the sequence which encodes the immunogenic polypeptide), whilst in other embodiments the presence of tetracycline will serve to enhance expression of the tet O-linked sequence.

Those skilled in the art will appreciate that a number of analogues of tetracycline are known, which can readily be substituted for tetracycline in the method of the invention. Indeed, certain analogues may actually be preferred to tetracycline, as they may have higher binding affinities (for the tetracycline-sensitive polypeptide) and so exert a regulatory effect at lower concentrations. A tetracycline analogue may be understood to refer to a molecule which is structurally related to tetracycline and which binds to the Tet repressor with a Kₐ of at least about 10⁶ M-¹, preferably 10⁹ M-¹ or greater. Preferred analogues are doxycycline and anhydrotetracycline. Other analogues include minocycline, oxytetracycline, chlorotetracycline, epioxytetracycline and cyanotetracycline. Other analogues of tetracycline are described by Hlavka & Boothe ("The Tetracyclines" in "Handbook of Experimental Pharmacology 78, Blackwood *et al*, (eds.) Springer Verlag 1985).

The tet operator (tet O) sequence is now well-known to those skilled in the art. For a review, the reader is referred to Hillen & Wissmann (1989) in Protein-Nucleic Acid Interaction. "Topics in Molecular and Structural Biology", eds. Saenger & Heinemann, (Macmillan, London), Vol. 10, pp 143-162. Typically the nucleic acid sequence encoding the leukocyte-activating molecule will be placed downstream of a plurality of tet O sequences: generally 5 to 10 such tet O sequences are used, in direct repeats.

Conveniently, the tet O sequences will be fused substantially adjacent (i.e. within 100bp, preferably within 50 bp) to a "minimal" (i.e. enhancerless) eukaryotic promoter (such as the minimal CMV immediate early promoter, described previously [Gossen & Bujard 1992 Proc. Natl. Acad. Sci. USA **89**, 5547]), such that binding of a transactivating tetracycline-sensitive polypeptide to the tet O sequence will cause enhanced expression of the tet O-linked coding sequence.

Constructs particularly suitable for introduction of the sequence encoding the leukocyte-activating molecule and/or a drug-regulatable promoter are well-known and have been disclosed previously (e.g. Baron *et al*, 1995 Nucl. Acids Res. **23**, 3605-3606; Schultze *et al*, 1996 Nature Biotechnology **14**, 499-503). A particularly preferred construct (where tetracycline, or an analogue thereof, is the regulatory drug), found to allow for very high levels of regulation of expression of tet O-linked sequences, is disclosed by Mohammadi *et al.*, (1997 Gene Therapy *4*, 991-997; 1998 Gene Therapy 5, 76-84) and by Alvarez-Vallina *et al.*, (J. Immunol. 5889-5895).

The method is desirably one wherein regulation of expression of the leukocyte-activating molecule enables the leukocyte preferentially to react with target cells expressing a relatively high density of leukocyte-stimulating molecules compared to non-target cells expressing a relatively low density of leukocyte-stimulating molecules.

The leukocyte is preferably a monocyte, macrophage or a lymphocyte, most preferably a T lymphocyte. For T lymphocytes (or T cells), the leukocyte-stimulating molecule is conveniently a "foreign" antigen or a tumour-associated antigen (e.g. CEA), whilst the leukocyte-activating molecule preferably comprises the intracellular (cytoplasmic) signalling domain of at least one of the chains of the T cell receptor (TCR) CD3 complex or the intracellular signalling domain of a co-stimulatory molecule such as CD28, CD4 or CD8.

Many tumour-associated antigens are not unique to tumour cells: they are expressed at relatively high densities on the surface of tumour cells, but may also be expressed at lower density on the surface of certain non-tumour cell types in a patient. Accordingly, many therapeutic methods which attempt to target cytotoxic agents, or to direct immune responses, to tumour cells via a tumour associated antigen often result in collateral damage to non-tumour cells.

The present specification discloses a method of targeting therapeutic effects specifically to cells expressing a high density of, for example, tumour associated antigen. Expression of the leukocyte-activating molecule on the surface of the leukocyte (which delivers or mediates the therapeutic effect) can be regulated, thereby altering the density of leukocyte-stimulating molecule (e.g. tumour-associated antigen) needed to reach the threshold level of interaction at which the leukocyte becomes activated.

For example, T lymphocytes can be transformed with a nucleic acid sequence directing the tetracycline-sensitive expression of a chimeric TCR molecule having specific binding activity for a tumour associated antigen. Adjustment of the amount of tetracycline (or analogue thereof) administered to the patient can be made, such that the density of tumour-associated antigen on tumour cells is sufficient to cause activation of the T cell, whilst the density of the tumour-associated antigen on non-tumour cells is too low, minimising collateral damage. The desired adjustment of tetracycline concentration in the patient can be made by trial-and-error, by analysis of clinical symptoms or markers. Other regulatable systems which might be used to regulate the expression of the leukocyte-activating molecule are disclosed, for example by Miller & Whelan (1997 Hum. Gene Ther. **8**, 803-815).

In certain embodiments therefore, where the leukocyte-stimulating molecule is a tumour-associated antigen, it will be expressed at relatively high density on a tumour target cell and expressed at relatively low density on non-tumour, non-target cells, such that performance of the method of the first aspect of the invention results in a leukocyte which is able preferentially to recognise and attack tumour target cells but which substantially does not attack non-tumour cells.

The leukocyte-activating molecule is conveniently membrane bound, typically comprising an intracellular (cytoplasmic) domain, a transmembrane domain, and an extracellular domain. Desirably the extracellular domain has binding specificity for the leukocyte-stimulating molecule. The leukocyte-stimulating and/or the leukocyte-activating molecule may be monomeric or polymeric (e.g. dimerie, trimeric etc).

Advantageously the leukocyte-activating molecule will be a chimeric polypeptide. For example, the leukocyte may be transformed with a sequence directing the drug-regulatable expression of a TCR molecule, or of a chimeric TCR molecule which comprises at least one functional domain (preferably the cytoplasmic signalling domain) of the TCR molecule. Where the leukocyte-activating molecule is a chimeric TCR, the transmembrane domain (necessary to anchor the molecule on the surface of the T cell) may be from the wild type TCR molecule, or may comprise a transmembrane (TM) domain from any other molecule which is present on the surface of a eukaryotic cell, or may be a "synthetic" non-naturally occurring transmembrane domain. Preferably the TM domain is that from a member of the immunoglobulin family of polypeptides.

In preferred embodiments the extracellular domain comprises or is derived from an antibody or an antigen-binding fragment thereof (such as an scFv, Fab or the like). In particular embodiments the leukocyte-activating molecule comprises an extracellular domain having binding affinity for a leukocyte-stimulating molecule which is a tumour-associated antigen (e.g. as described by Pardoll, reference 7).

Other embodiments of the invention (e.g. relating to antigen density-specific activation of B lymphocytes) will be apparent to those skilled in the art. In a first aspect the invention provides a leukocyte transformed with a nucleic acid sequence which expresses a leukocyte-activating molecule in a manner sensitive to the concentration of an exogenous agent, the leukocyte being activated by an interaction between the leukocyte-activating molecule and a leukocyte-stimulating molecule present on the surface of the cell, wherein the leukocyte is differentially reactive to different densities of leukocyte-stimulating molecules and distinguishes between target cells with relatively high densities of leukocyte-stimulating molecules and non-target cells with relatively low densities of leukocyte-stimulating molecules. The leukocyte is typically one which has been treated by the method disclosed above.

In a second aspect the invention provides a composition for use in a therapeutic method, the composition comprising a plurality of leukocytes in accordance with the first aspect of the invention defined above, and a physiologically acceptable diluent. The invention further provides, in a third aspect, a method of making such a composition, the method comprising: obtaining a sample of leukocytes, preferably obtained from a subject to be treated; transforming the leukocytes with a nucleic acid sequence which directs the expression of a leukocyte-activating molecule in a manner sensitive to the concentration of an exogenous agent; and mixing the leukocytes with a physiologically acceptable diluent (such as saline, phosphate-buffered saline, tissue culture medium, etc).

Additionally the specification discloses the use of compositions according to the invention for treating a human or animal subject, the use comprising preparing a composition as defined immediately above, administering the composition to the subject and, if necessary, administering an exogenous substance to the subject so as to alter the level of expression of the leukocyte-activating molecule in the administered leukocytes.

The various aspects of the invention find application in a number of fields, but particularly in or relating to treatment of tumours and other neoplastic diseases.

Typically, the relevant nucleic acid sequences are introduced into the leukocyte in *vitro.* Numerous methods of introducing nucleic acid sequences into eukaryotic cells are known, including transfection, transduction, electroporation, cell fusion and the like. Any of these methods may be used in the present invention, and may generally be referred to as transformation.

Similarly, methods of introducing the transformed leukocyte into a subject are well known. Conveniently this is done by infusion or injection into the subject's bloodstream. Typically between 10⁵ and 10⁸ cells (preferably between 10⁶ and 10⁷) will be introduced into the subject's bloodstream. It will be appreciated that introduction of foreign cells into a subject is likely to create an immune response against foreign antigens on the cell, so generally the leukocytes will be tissue-matched with the recipient subject. Most conveniently, the cells will be autologous cells originally obtained from the subject (e.g. from peripheral blood, or from bone marrow), transformed *in vitro* with the relevant nucleic acid sequences, and then re-introduced into the subject. Typically, the *in vitro* stages of the method will generally comprise a selection process to select those cells successfully transformed, and or a growth stage, to increase the numbers of transformed cells. Methods of selection and growth of cells are well known to those skilled in the art and form no part of the present invention.

It is possible that the leukocyte-activating molecule may be immunogenic in the subject. Where the invention involves regulating the expression of a potentially immunogenic leukocyte-activating molecule, it will be desirable to cause a delay in expression of the molecule after the cell is introduced into the subject, for reasons explained in greater detail below. Accordingly, the cell is generally transferred from conditions *in vitro* in which expression of the immunogenic polypeptide is fully repressed, to conditions *in vivo* in the subject in which the expression of the leukocyte-activating molecule is no longer down-regulated, However, the invention is such that it takes a significant period (typically 2 to 10 days, preferably 4 days or longer) for the cell to move from the fully-repressed state to the fully-expressed state.

The cell may be in a state in which expression of the leukocyte-activating molecule is fully repressed *in vitro* by exposure to appropriate (non-toxic) concentrations of the regulatory drug (e.g. tetracycline or an anaogue thereof), and when introduced into a regulatory drug-free subject eventually enters a state in which the leukocyte-activating molecule is expressed. Alternatively, as described above, because of the versatility of the TRS and the variants thereof and of other drug-regulatable promoter systems available to the person skilled in the art, the leukocyte may be maintained in a fully-repressed state *in vitro* in the absence of regulatory drug, and then introduced into a subject receiving appropriate doses of the regulatory drug, so as to cause the leukocyte-activating molecule to be expressed (after an appropriate delay).

Generally, although not essentially, it is preferred that the presence of regulatory drug inhibits expression of the leukocyte-activating molecule as subsequent removal of the cell from regulatory drug exposure normally gives a longer lag phase or delay before induction of expression of the leukocyte-activating molecule.

The efficiency of tumour attack by the introduced leukocytes may be enhanced by causing the cells to express targeting entities on their cell surface (see, for example, Eshar *et al,* 1993 Proc. Natl. Acad. Sci. USA **90,** 720; Hwu *et al*, 1993 J. Exp. Med. **178,** 361; Stancovski *et al*, 1993 J. Immunol. **151,** 6577; and Brocker *et al*, 1996 Eur. J. Immunol. **26,** 1770). Once within the tumour, the leukocytes can exert their therapeutic effect (e.g. cytotoxic action, or recruitment of macrophages and lymphocytes).

Any solid tumour, accessible by blood-borne leukocytes, could be available for treatment by the method of the invention. Conveniently the leukocyte introduced into the subject also comprises a cell-surface component which targets the leukocyte to a marker expressed on the surface of the tumour cells. These may be, for example, chimeric "T body" targeting components, known to those skilled in the art (see disclosures of Eshar *et al,* Hwu *et al*, Stancovski *et al*, and Brocker *et al*, cited above).

However, targeting of the introduced leukocytes to the tumour takes some time. Thus, expression of the immunogenic polypeptide during this time is preferably avoided as it may cause: (a) collateral damage to non-malignant cells and/or (b) interaction of the immunogenic protein with components of the subject's immune system (especially circulating antibodies). This latter point is particularly pertinent if repeated administrations of the leukocytes is required, as this would cause efficient induction of immune responses to the immunogenic polypeptide, which would tend to interact with the administered leukocytes and prevent them from reaching the target tumour. These problems can be overcome by the method disclosed herein, in which desirably the potentially immunogenic leukocyte-activating molecule is only allowed to become expressed at high levels after a significant time delay, by which point the administered leukocytes will have penetrated the target tumour, thereby preventing interception by the immune system and minimising collateral damage to non-malignant cells. Accordingly, the method disclosed herein can be performed in a subject which has already made an immune response to the leukocyte-activating molecule and who may have, for example, circulating antibodies which react with, or immunocompetent memory cells specific for, the leukocyte-activating molecule.

It may be desirable to modify the components of the drug-regulatable promoter system employed in the method so as to reduce their immunogenicity (e.g. by modifying any DNA-binding protein so as to remove certain epitopes). Conveniently, if present, the DNA-binding protein will comprise a nuclear localization signal (NLS), so as to minimise the amount which might become presented to the subject's immune system. Other useful techniques are disclosed in WO 96/01313.

Another advantage conferred by the invention is, of course, the ability to control the concentration of exogenous agent to which the leukocyte is exposed, thereby controlling the level of expression of leukocyte-activating molecule in the leukocyte. This in turn controls the density of leukocyte-stimulating molecules required to cause activation of the leukocyte. The inventors have found that by controlling the amount of leukocyte-activating molecule on the leukocyte, the leukocyte can be made to require a threshold density of leukocyte-stimulating molecules on a cell for the leukocyte to become activated: if the cell does not possess a sufficient density of leukocyte-stimulating molecules, it will not cause the leukocyte to become activated and the cell will not be attacked by the leukocyte. Conversely, if the cell has a density of leukocyte-stimulating molecules above the threshold density, leukocytes encountering the cell will become activated and attack the cell.

In most embodiments, the regulatory drug will be tolerated when administered to a subject. Accordingly, if desired, in those situations where the transformed cells are exposed *in vitro* to the regulatory drug so as to inhibit expression of the leukocyte-activating molecule, the regulatory drug may also be administered to the patient before, during or after introduction of the transformed cells. This may be desirable for prolonging the delay interval, or for exposing the cells to intermediate concentrations of regulatory drug so as to regulate expression of the leukocyte-activating molecule or, if the patient should experience an adverse reaction to the expression of the leukocyte-activating molecule, the regulatory drug could be administered in a sufficient dose to prevent further expression thereof.

Methods of administration to the subject of the regulatory drug (if required) are well known to those skilled in the art and include oral administration (in any form), slow-release pellets and implantation of a diffusion pump, in addition to more conventional methods such as injection, infusion, inhalation or ingestion. As an example, if tetracycline or a tetracycline analogue is administered to a subject, the dosage is conveniently adjusted to achieve a serum concentration between about 0.05 and 1.0µg/ml.

In this way, the threshold density of leukocyte-stimulating molecules required for activation of the leukocyte will depend on the concentration of exogenous agent (or regulatory drug) to which the leukocyte is exposed. Thus, by adjusting the concentration of exogenous agent to the desired level, transformed leukocytes introduced into the subject can be made to distinguish between target cells (e.g. tumour cells) expressing a relatively high density of leukocyte-stimulating molecule (e.g. tumour-associated antigen) and non-target cells (e.g. non-tumour cells) expressing a relatively low density of leukocyte-stimulating cells. In this way, only target cells will cause the leukocytes to become activated, thereby minimising "collateral damage" to non-target cells.

The invention will now be further described by way of illustrative example and with reference to the accompanying drawings, in which:
Figures 1A and 1B are schematic representations of nucleic acid constructs referred to in the example below;
Figures 2A, 2B and 3 show representative FACS data;
Figure 4 is a graph showing expression of a chimeric polypeptide (as a percentage of expression in control cells) against time;
Figures 5A and 5B are bar charts showing the levels of IL-2 production (in picograms/ml) by T lymphocytes exposed to various concentrations of tetracycline or the tetracycline analogue, doxycycline;
Figure 6A is a graph of Mean Fluorescence Intensity against [Tetracycline] in ng/ml, showing expression of a chimeric TCR leukocyte-activating molecule regulated by concentration of an exogenous agent;
Figure 6B shows the FACS profiles of two leukocyte clones;
Figure 7A is a graph of OD (at 450nm) against concentration of NIP₁₀-BSA (in ng/ml) used to coat a microtitre plate;
Figure 7B is a graph of T cell activation as measured by IL-2 production (pg/ml), against concentration of NIP₁₀-BSA (in ng/ml) used to coat a microtitre plate;
Figure 7C is a graph of % viable cells against concentration of NIP₁₀-BSA (in ng/ml) used to coat a microtitre plate;
Figure 7D is a graph of % Annexin V binding against concentration of NIP₁₀-BSA (in ng/ml) used to coat a microtitre plate;
Figures 8A and 8B are cartographic maps of IL-2 production (pg/ml) at different target antigen (NIP₁₀-BSA) densities and different chTCR densities (as measured by mean fluorescence intensity, MFI).
Figure 9 shows the FACS profiles of HeLa S3 cells with (1-4) or without ("con") labelling with NIP-containing hapten, followed by staining with an anti-NIP antibody scFv fragment (B1.8) and an FITC-conjugated goat antiserum to mouse λ-chain; and
Figure 10 is a series of three panels showing IL-2 production (pg/ml) by NIP-responsive T cells following stimulation by HeLa S3 target cells coated with NIP-containing hapten at 1, 5 or 10 µg/ml (Figs. 10A, B and C respectively) at different effector: target ratios. The rearmost bar shows the results for T cells without tetracycline repression, the middle bar shows the results obtained with tetracycline-induced repression, and the foremost bar shows the unresponsiveness of negative control cells not expressing the NIP-specific chimeric T cell receptor.

### Detailed Description of an Embodiment of the Invention

### Example 1

The inventors have evaluated the utility of the tetracycline-controlled transactivator system as a means to temporally regulate the expression of a surface molecule in a human T cell line. Using a vector containing both the transactivator and the expression gene unit, we were able to generate stably transfected Jurkat T cell lines in which the expression of a chimeric TCR (chTCR) molecule could be efficiently regulated. Depending on the tetracycline analogue used and its concentration, the induction of chTCR can be reversibly repressed to a greater or lesser extent. Futhermore, we have shown that fully repressed T cells can not be activated to produce IL-2 via this chimeric receptor, indicating that reversible functional inactivation of redirected T cells is possible.

The time-course to repress gene expression to basal levels was significantly shorter than the time-course for gene expression to reach maximal levels after drug removal, and the delay in resumption of promoter activity varied considerably depending on the concentration of doxycycline (a tetracycline analogue) used for repression. The relevance of these data to current ideas on T cell mediated immunotherapy is discussed.

### Materials and Methods

**Reagents.** The mAbs used included SPvT3b (mouse IgG2a) (11) and YTH913.12 (rat IgG2b) (Serotec Ltd., Oxford, UK) specific for human CD3∈ and CD28 molecules respectively. For direct staining the following FITC-conjugated antibodies were used: UCHT-1 (anti-CD3∈, a mouse IgG1 Serotec; goat polyclonal antisera to mouse λ-light chain (Southern Biotechnology Associates, Inc, Birmingham AL); and goat polyclonal antisera to mouse IgG (γ-chain specific) (Sigma Chemical Co., St. Louis, MO). Bovine serum albumin (BSA) was conjugated with 4-hydroxy-5-iodo-3-nitrophenyl acetyl (NIP) (Cambridge Research Biochemicals, Northwich, UK) in a molar ratio of 10:1 (NIP₁₀-BSA) (12). Tetracycline hydrochloride (Sigma) was dissolved at a concentration of 0.5 mg/ml in culture medium. Doxycycline hydrochloride ("Dox") (Sigma) was dissolved in 0.02N HCl at a concentration of 1 mg/ml and further diluted in culture medium. The antibiotic solutions were freshly prepared on the day of use and diluted to the appropriate concentrations.

*Vector Construction.* Plasmids pUHD 15-1, containing the tTA transactivator gene transcribed from the human CMV immediate early (CMV IE) promoter/enhancer, and pUHD 10-3, containing a tTA-responsive promoter (TRP, heptamerized tetO sequences (TetO)7 fused to a human CMV immediate early minimal promoter [PhCMV*-1]), were kindly provided by H. Bujard (9). Plasmid pCS was constructed by removing a 1308 bp *Sal* I fragment, containing the CMV IE promoter, the multiple cloning site and the SV40 polyadenylation signal, from pCEP4 backbone (Invitrogen, San Diego, CA). A 6723 bp *Nru* I-*Cla* I digested fragment from pCS was blunt ended with Klenow (Cambio, Cambridge, UK) and inserted into the *Xho* I site of plasmid pUHD 15-1 following the treatment of this site with Klenow and calf intestinal alkaline phosphatase (CIP, Boehringer Mannheim GmbH, Germany). The resulting plasmid containing both the tTA and the hygromycin transcription units in opposite directions was designated pCRAZY.

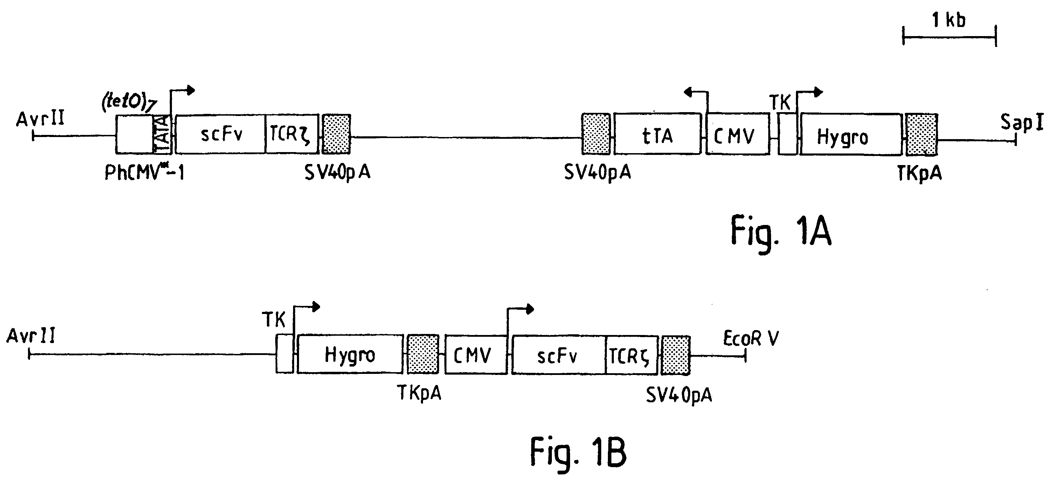

A chimeric NIP-specific scFv-TCR ζ molecule was constructed as described previously (12) and cloned into the plasmid pUHD 10-3. To do this the *Hind* III site from pUHD 10-3 was removed by cleavage with *Hind* III followed by Klenow fill-in and blunt-end ligation resulting in pLAV5. To construct pLAV6, a 1342 bp *EcoR* I-*Xba* I fragment derived from the plasmid pVACl.aNIP.TCR ζ (described in reference 12), containing a human VH1 leader sequence and a chimeric NIP-specific TCR ζ molecule, was cloned into the *Eco*R I-*Xba* I polylinker site of pLAV5. The 91 bp *EcoR* I-*Hin*d III fragment containing a Rous Sarcoma virus (RSV) promoter partial sequence was removed from pLAV6 by digestion with *Eco*RI and *Hind* III, Klenow fill-in and blunt end ligation to produce plasmid pLAV7. A polylinker containing restriction sites unique to the vector construct , *Hin*d III-*Bg*l II-*Eco*R V-*Cla* I, (5826: 5'-CATCGATCGAACTGATATCAGCAGATCTCAGAAGCTTAAT-3' Seq. ID No. 1 and 5827: 5'-ATTAAGCTTCTGAGATCTGCTGATATCAGTTCGATCGATGACGT-3' Seq. ID No. 2) was ligated into the *Ssp* I-*Aat*II site of pLAV7 resulting in pLAV8. To construct a single plasmid with both the tTA transactivator gene and the chimeric NIP-specific scFv-TCR ζ gene under the control of TRP in antisense orientation (relative to the tTA transcription unit) the plasmid pCRAZY was digested with *Xmn* I and a *Bg*l II linker (New England Biolabs, Inc., Beverly, MA) was introduced at this site. After digestion with *Bg*l II and *Hind* III a 9386 bp fragment was inserted into the *Bgl* II*-Hin*d III site of pLAV8. The resulting plasmid was designated pLAV12 (Fig. 1A).

*Cell Culture and Transfections.* The Jurkat T cell line (clone E6-1) was maintained in RPMI 1640 supplemented with 10% foetal calf serum (FCS), 2 mM L-glutamine, 100 U/ml penicillin, 100 µg/ml streptomycin and 25 mM HEPES buffer (all from GIBCO-BRL, Gaitersburg, MD), referred to as complete medium (CM). To generate stable cell lines Jurkat cells were transfected with linearized plasmid DNA (10 µg) by electroporation, at 250 mV and 960 µF, as described previously (13). Transfectants were selected in CM supplemented with 0.4 mg/ml of hygromycin B (Calbiochem, San Diego, CA). Stable cell lines were established after 3-4 weeks and analyzed by FACS for expression of the chTCR. To select a population of chTCR ζ expressing cells, stable Jurkat cells transfected with the linearized pLAV12A or pCEP4.aNIP.TCR ζ derived plasmid fragments were FACS sorted (see below). The resultant populations were cloned twice by limiting dilution and screened for protein expression by flow cytometry.

*Flow Cytometry and Cell sorting*. Expression of cell surface proteins was performed by standard direct immunofluorescence as described (14) using saturating amounts of FITC-conjugated antibodies. Dead cells were excluded from analysis using a combination of propidium iodide and forward light scatter. Appropriate FITC isotype-matched irrelevant Abs were used in all experiments. The samples were analysed with a FACScan® (Becton Dickinson, Mountain View, CA). A minimum of 20,000 cells was analysed for each sample. Subsequent re-analysis of data was performed using the CELLQuest software (version 1.2) (Becton Dickinson). Additionally, cells stained with FITC-conjugated goat antisera to mouse λ light chain were sorted under sterile conditions on a cell sorter (FACScalibur, Becton Dickinson).

*IL-2 Release Assay*. The cells were pre-incubated at a concentration of 5x10⁵/ml for 48 hours in CM in the absence or presence of tetracycline or doxycycline at the indicated concentrations. Subsequently the cells were washed, counted and stimulated (10⁵/well) in triplicate with plastic-immobilised NIP10-BSA conjugates (iNIP10-BSA) or plastic-immobilised anti-CD3∈ mAb (anti-CD3) in fresh CM alone or in the presence of the drugs (12). The plates were incubated at 37°C in 5% CO₂. After 20 hours supernatants were harvested and assayed for IL-2 activity using an ELISA kit (Genzyme Diagnostics, Cambridge, MA).

### Results

### Design of the tet-regulatable Vector.

To determine if the expression of a chTCR could be pharmacologically regulated in T cells, the inventors constructed the plasmids pLAV12 and pCEP4.aNIP.TCR ζ (Figure 1 and Alvarez-Vallina *et al,* 1997 J. Immunol. **159**, 5889). Figures 1A and 1B are schematic maps of representative plasmid fragments used in the experiments showing the predicted structure after integration into the host genome: (A) pLAV12 and (B) pCEP4.aNIP.TCRζ, respectively. The direction of transcription is indicated by arrows.

Both constructs encode a chimeric TCR molecule that has been described previously (12), and comprises the antigen combining site of the hapten-specific (NIP) mAb B1.8 (15) fused to the transmembrane and cytoplasmic regions of the human TCR ζ chain (16). pLAV12 (Fig. 1A) is a tetracycline-regulatable construct containing all the components of the TRS with the tTA gene under the control of the constitutive CMV IE promoter and the gene coding for the chTCR inserted under the control of the tTA-responsive promoter. In an attempt to reduce potential cis-regulatory enhancement of TRP activity, due to proximity to the other enhancer and promoter elements present on the vector, the tTA-response cassette was separated from the other transcriptional units by a 2500 bp fragment containing the pUC derived ColE1 origin of replication and the β-lactamase gene (Fig. 1A). In the control construct pCEP4.aNIP.TCRζ the chTCR molecule was under the control of the constitutive CMV IE promoter (Fig. 1B).

Both constructs encode a hygromycin resistance marker gene transcribed by a constitutive promoter. To promote the stable integration of these DNA constructs in the designed configuration in transfected T cells, a linearized *Avr* II-*Sap* I 9975 bp DNA fragment (Fig. 1A) from plasmid pLAV12 and a linearized *Avr* II-*Eco*RV 7551 bp fragment (Fig. 1B) derived from plasmid pCEP4.aNIP.TCRζ, both lacking the EBV replication origin and the EBNA-1 gene (Fig. 1), were used to transfect Jurkat cells.

### tTA-dependent Expression of the Chimeric scFv Gene Construct.

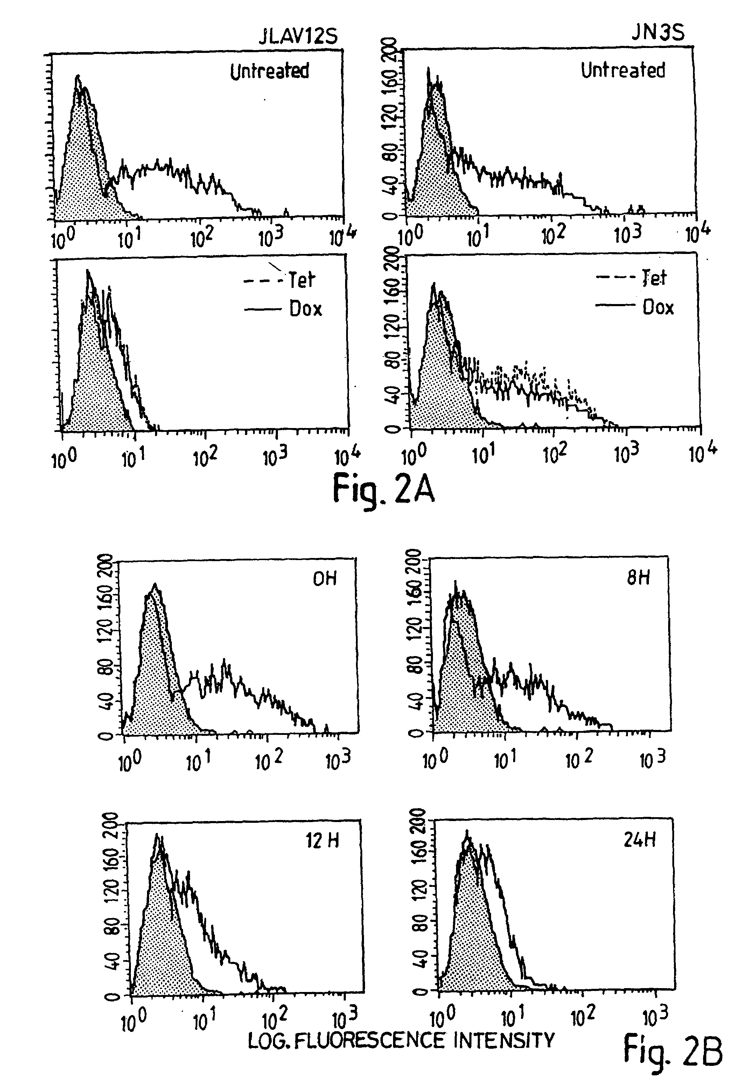

Jurkat E6-1 cells were transfected with linearized fragments of pLAV12 and pCEP4.aNIP.TCRζ. To select for higher expression of the chTCR, the stably transfected hygromycin-resistant cells were FACS sorted after staining with a FITC-labelled goat anti mouse λ-light chain antiserum. Most of the isolated pLAV12 transfectants (JLAV12S) and pCEP4.aNIP.TCRζ transfectants (JN3S cells), expressed the chTCR although there was considerable heterogeneity in the absolute levels of expression (Fig. 2A, 2B).

Figure 2 shows representative results confirming the regulation of the chTCR gene expression by tetracycline analogues. In Figure 2A, stable transfected uncloned JLAV12S (left hand side) and JN3S Jurkat (right hand side) cell populations were cultured for 48 hours in tetracycline-free medium (CM, upper row of panels) or in the presence of 1 µg/ml of Tet (broken line) or Dox (solid line)(lower row of panels) and the surface expression of chTCRs was examined after staining with FITC-conjugated goat antisera to mouseλ light chain. Figure 2B shows a timecourse of inactivation of chTCR gene expression in JLAV12S cells zero hours (top left), 8 hours (top right), 12 hours (bottom left) or 24 hours (bottom right) after addition of Dox at 1 µg/ml. In both Figures 2A and 2B negative controls (FITC-conjugated goat antisera to mouse IgG) are overlaid (filled curve). The fluorescence channel number is plotted along the x axis, and the y axis represents the relative cell number.

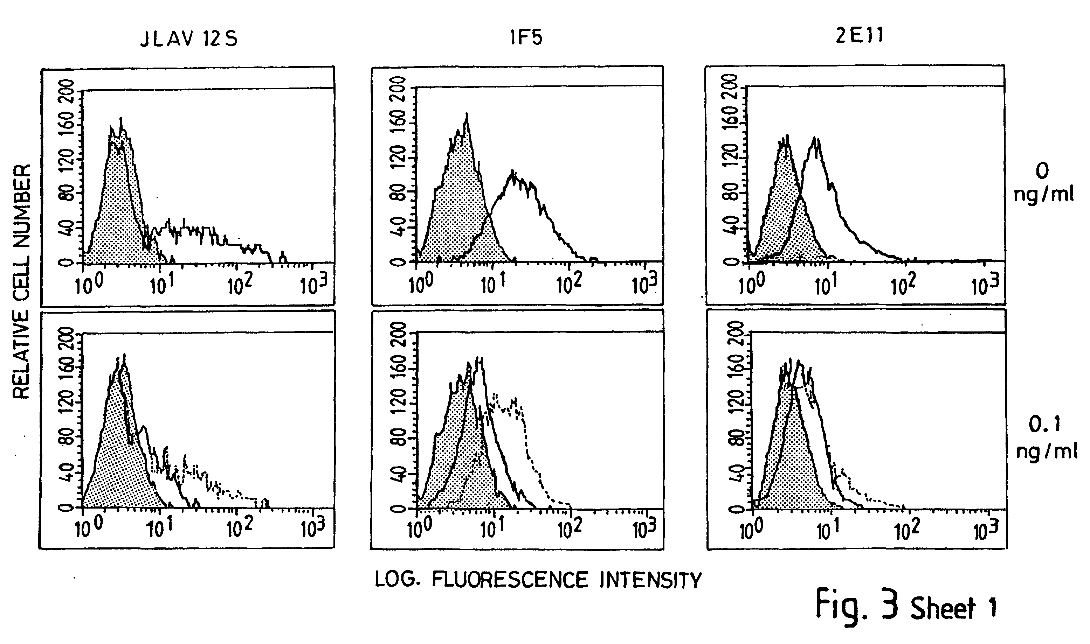

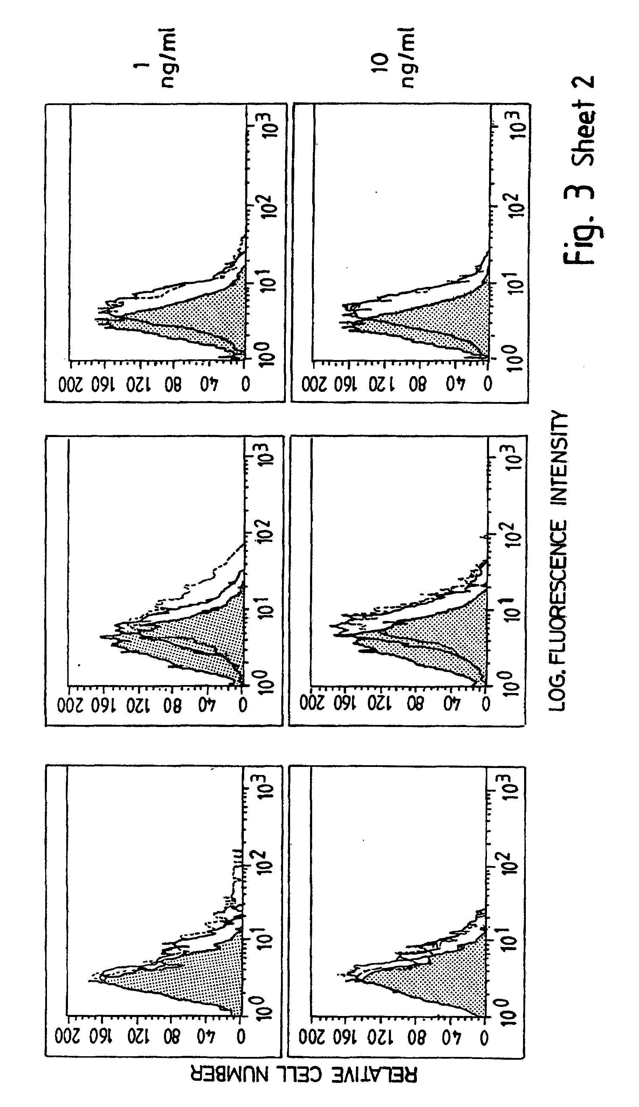

The selected population of pLAV12 transfectants (JLAV12S) was then cloned by limiting dilution and two subclones expressing the chTCR at low (2E11) and intermediate (1F5) levels (Figure 3) were selected for further study.

### Regulation of the Chimeric scFv-TCRζ Gene Expression.

To determine whether the expression of the chTCR could be suppressed by tetracyclines, JLAV12S and JN3S cells (5x10⁵/ml) were incubated for 48 hours in the presence of 1µg/ml of tetracycline (Tet) or its analogue doxycycline (Dox). At this concentration most of surface chTCRs (90%) were down-regulated in JLAV12S cells, but were not affected in JN3S cells (Figure 2A). Also, surface staining with anti-CD3∈ mAbs demonstrated that the amount of TCR/CD3 complex remained constant in both cell populations (not shown). No changes in cell viability were observed at this concentration, assayed using trypan blue staining (data not shown).

To study the time-course of inactivation of gene expression, JLAV12S cells were analysed at different times after addition of the antibiotics at 1 µg/ml (Fig. 2B). A slight reduction was observed within 8 hours of exposure to the drugs and maximum repression was achieved within 24 hours, when the expression of the chTCR fell to less than 10 percent of the level observed in the absence of tetracyclines at the same time point (Figure 2B). Similar results were observed in the 1E5 and 2E11 clones where the level of chTCR was reduced to about 10% (1E5) and 20% (2E11) of its maximal expression (Figure 3). The time-course of gene repression was very similar in response to both antibiotics (Tet or Dox) in all analysed populations. It is important to note that the percentage of cells in which tetracyclines did not down-regulate the expression of chTCRs was < 0.5%, not affecting the overall regulation in the whole population of JLAV12S cells (Figure 2A).

A dose-response curve of gene repression was determined using different concentrations of Tet or Dox. After 48 hours of treatment, the cells were harvested and the expression of the chTCR was studied by FACS analysis. Typical results are shown in Figure 3.

Stably transfected uncloned (JLAV12S, left hand column) and cloned (1F5, middle column; and 2E11, right hand column) Jurkat cell populations were cultured for 48 hours in the presence of different concentrations (0ng/ml top row, 0.1ng/ml second row, 1ng/ml third row, and 10ng/ml bottom row) of Tet (broken line) or Dox (solid line) and the surface expression of scFv-TCR ζ molecules was examined. Negative controls (FITC-conjugated goat antisera to mouse IgG) are overlaid (filled curve). The fluorescence channel number is plotted along the x axis, and the y axis represents the relative cell number.

Referring to Figure 3, in both clonal populations (1E5 and 2E11) the expression of chTCRs was maximally repressed at 100 pg/ml (0.1ng/ml) of Dox, with no further increment in the level of repression at higher concentrations. Partial activity of the TRP was observed in the concentration range of 1 pg/ml to 100 pg/ml of Dox (data not shown). In JLAV12S cells maximal repression was observed at Dox concentrations greater than or equal to 1 ng/ml. For tetracycline maximal repression occurred, in all the analysed cell lines, at concentrations greater than or equal to 10 ng/ml. The induction of the chTCR gene was only partially repressed at tetracycline concentrations of 100 pg/ml to 1 ng/ml.

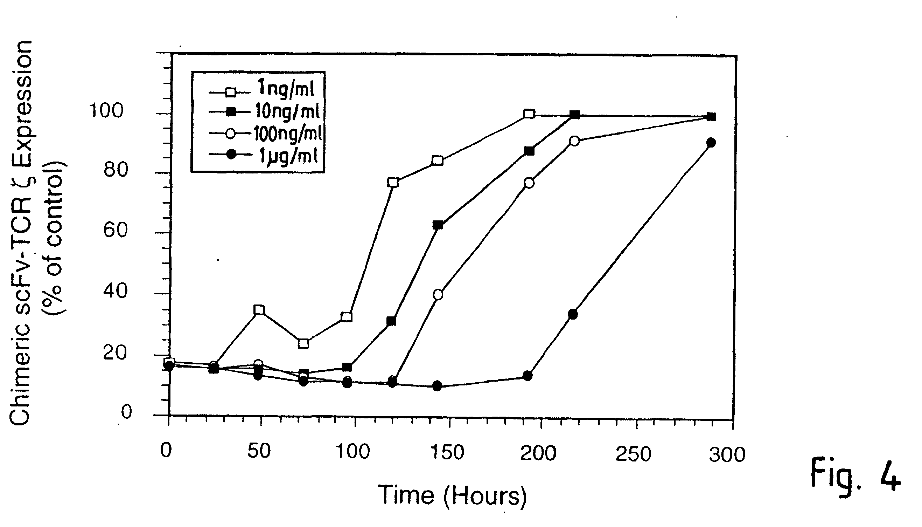

To study the kinetics of recovery of TRP-driven gene expression after withdrawal of tetracyclines, stable transfected uncloned JLAV12S cells were cultured for 48 hours in the presence of different concentrations of Dox (1 ng/ml to 1 µg/ml). After three washes the cells were incubated (5x10⁵/ml) in tetracycline-free CM in new plates and the surface expression of chTCRs was examined every 24-48 hours after staining with FITC-conjugated goat antisera to mouse λ light chain. The results are shown in Figure 4. Similar experiments were also performed with 1F5 cells (data not shown). 100% values correspond to the median fluorescence of control untreated cells after staining with FITC-conjugated goat antisera to mouse light chain. The values are the percentage of chimeric TCRζ molecules expressed on tetracycline treated cells from each cell population as compared with the amount of chimeric TCRζ molecules on control untreated cells (as 100%). Referring to Figure 4, after treating the cells with 1 µg/ml of Dox (filled circles), recovery of chTCR expression was not apparent at 192 hours after removal of the drug and was first detected on the cell surface after 216 hours, with full recovery of expression after 288 hours. In contrast, the TRP remained repressed for only 24 hours after drug removal, when cells were pretreated with 1 µg/ml Tet, with maximal levels of chTCR expression being reached after 72 hours (data not shown). Treating the cells with lower concentrations of Tet (not shown) or Dox (1ng/ml - open squares, 10ng/ml - filled squares, 100ng/ml - open circles) resulted in earlier recovery of the activity of the TRP.

### Example 2

### Functional Study

It has previously been shown that the chTCR employed in this study is able to mediate specific recognition of its cognate antigen (NIP conjugated to BSA), soluble or plastic immobilized, resulting in the production of IL-2 by the transfected T cells (12). In the current study the inventors found consistently that stimulation of chTCR expressing cells (JN3S, JLAV12S, 1F5 and 2E11) with plastic immobilized NIP₁₀-BSA conjugates induced IL-2 secretion (data not shown). The level of IL-2 production varied between different transfectant cell populations, but in general it was similar to that observed in the same cell population in response to standardised stimulation with anti-CD3∈ mAb immobilized in microtiter wells (not shown).

Given that high concentrations of tetracycline have been shown to interfere with the process of T cell activation (17), the inventors studied the effects of increasing concentrations of Tet and Dox on the anti-CD3∈ induced IL-2 secretion of Jurkat cells. IL-2 secretion was unaffected by 100 ng/ml or lower concentrations of doxycycline but was inhibited by 25% at a doxycycline concentration of 1µg/ml (not shown). Tetracycline at concentrations lower than or equal to 1 µg/ml did not influence the anti-CD3∈ induced IL-2 secretion, which was similar to that observed in untreated cells (not shown). These results indicate that is possible to induce maximal TRP repression at concentrations of doxycycline more than 1000-fold lower than the threshold at which immunomodulating effects on human T cells are first manifest.

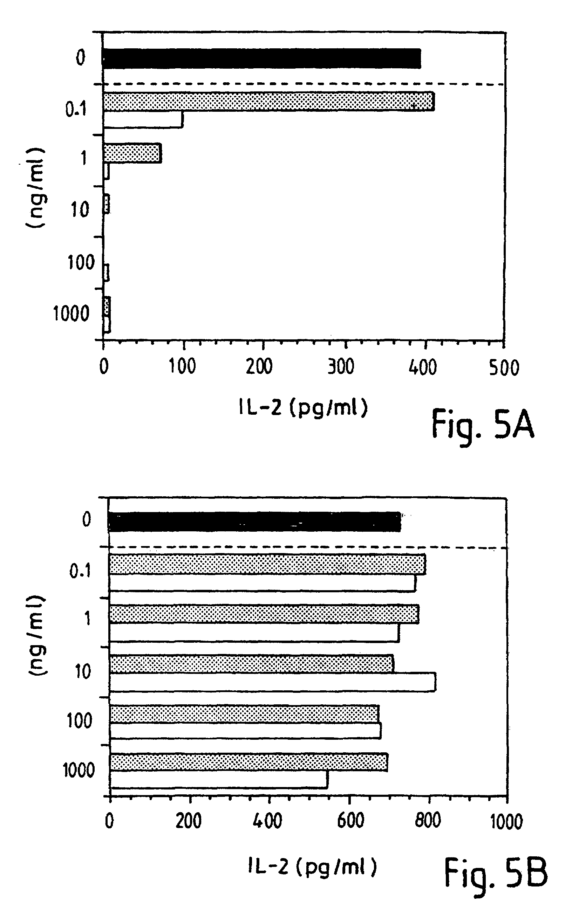

The inventors next determined whether tetracycline-mediated. suppression of the chTCR could induce a reversible state of antigen unresponsiveness in the transfected Jurkat cells. JLAV12S and JN3S cells were preincubated for 48 hours in increasing concentrations of Dox and Tet and then stimulated with immobilized NIP-BSA, following which their IL-2 production was measured. In situations of full TRP repression JLAV12S cells down-regulated 90% of surface chTCRs (see Figure 3), and were completely unresponsive to stimulation with iNIP10-BSA conjugates (see Figure 5A).

Figures 5A and 5B are bar charts showing IL-2 production (in pg/ml) by transfected JLAV12S (5A) or JN3S (5B) cells stimulated with iNIP10-BSA in the absence or presence of Tet or Dox. Cells were preincubated for a 48 hours period in the absence or presence of the drugs (at the indicated concentration in ng/ml), washed and stimulated (10⁵/well) with plastic immobilised NIP10-BSA conjugates (50 µg/ml) in fresh CM alone (solid bar) or in the presence of Tet (shaded bars) or Dox (open bars) at the indicated concentrations. One of two similar experiments is shown.

Down-regulation of about 75% of chTCRs was associated with low IL-2 production, whereas no inhibitory effect was observed when the level of chTCR expression was about 50% of that observed in absence of tetracycline (Figure 5A). These results indicate that the number of surface chTCR molecules expressed by JLAV12S cells in situations of full TRP repression is not enough to reach the activation threshold required for optimal T cell function. In contrast, when JN3S cells were stimulated with iNIP10-BSA conjugates, there was no inhibitory effect of tetracycline and of doxycycline at a concentration of less than 1 µg/ml (Figure 5B).

This example shows the feasibility of using regulated expression of leukocyte-activating molecules on the surface of leukocytes to render the leukocyte differentially sensitive to different densities of leukocyte-stimulating molecules (such as antigens) present on the surface of a target cell.

### Example 3

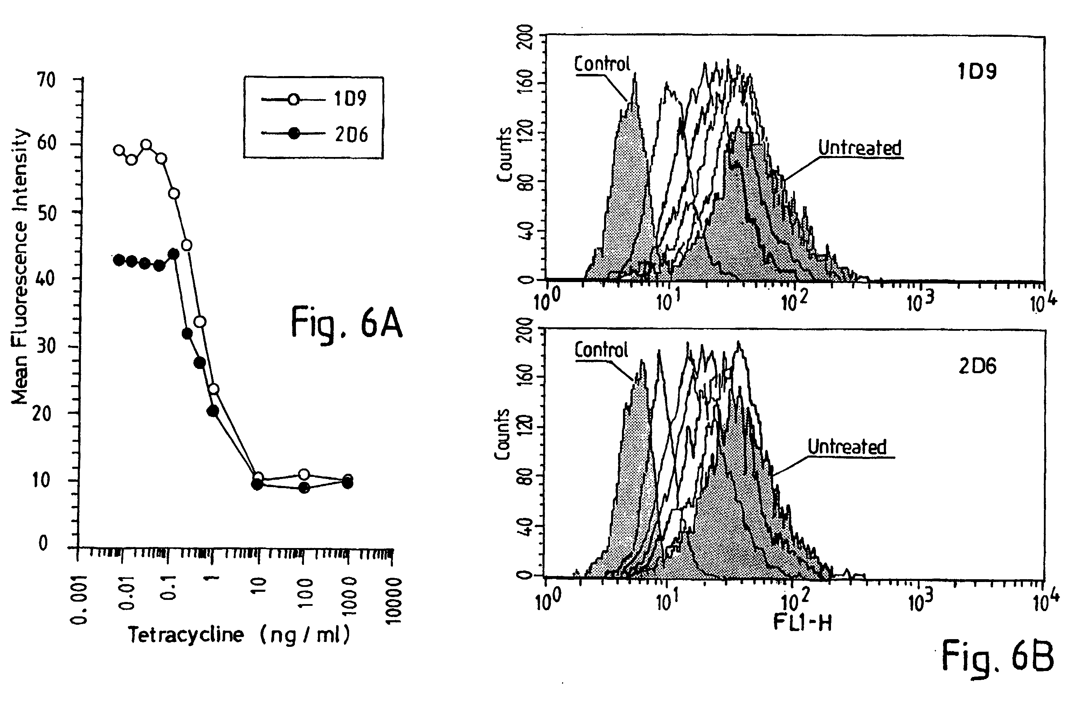

Jurkat E6-1 T cells were stably transfected with linearized pLAV12 and clones (1D9 and 2D6) with high-level tetracycline-repressible expression were selected, as described above. The system is optimal for receptor density studies because it allows transcriptional regulation of the chTCR expression without TCR engagement that could generate intracellular signals and modulate cell responsiveness (21). Using the appropriate range of tetracycline concentrations subtle changes in the level of the chTCR expression could be easily induced and stably maintained at a particular level by continuous culture in a fixed concentration of tetracycline (Fig. 6A and 6B).

Figure 6A shows the mean fluorescence intensity values of 1D9 (open circles) and 2D6 (filled circles) cells cultured for 48 h in the presence of different concentrations of tetracycline, after staining with FITC-conjugated antiserum to mouse λ light chain.

Figure 6B shows the FACS profiles of 1D9 (upper panel) and 2D6 (lower panel) cells cultured for 48 h in tetracycline-free medium (curve labelled "untreated") or in the presence of different concentratons of tetracycline in the 0.1 to 10 ng/ml range (open curves between "untreated" and "control" profiles). Negative controls (FITC-conjugated goat antiserum to mouse IgG) are overlaid (grey filled curve at left hand end of graphs).

It was found that clones 1D9 and 2D6 could be activated to secrete IL-2 upon antigen-mediated ligation of their chTCR by plastic immobilised NIP10-BSA conjugates (iNIP10-BSA). Microtitre plates were coated with NIP10-BSA, and checked, as follows;

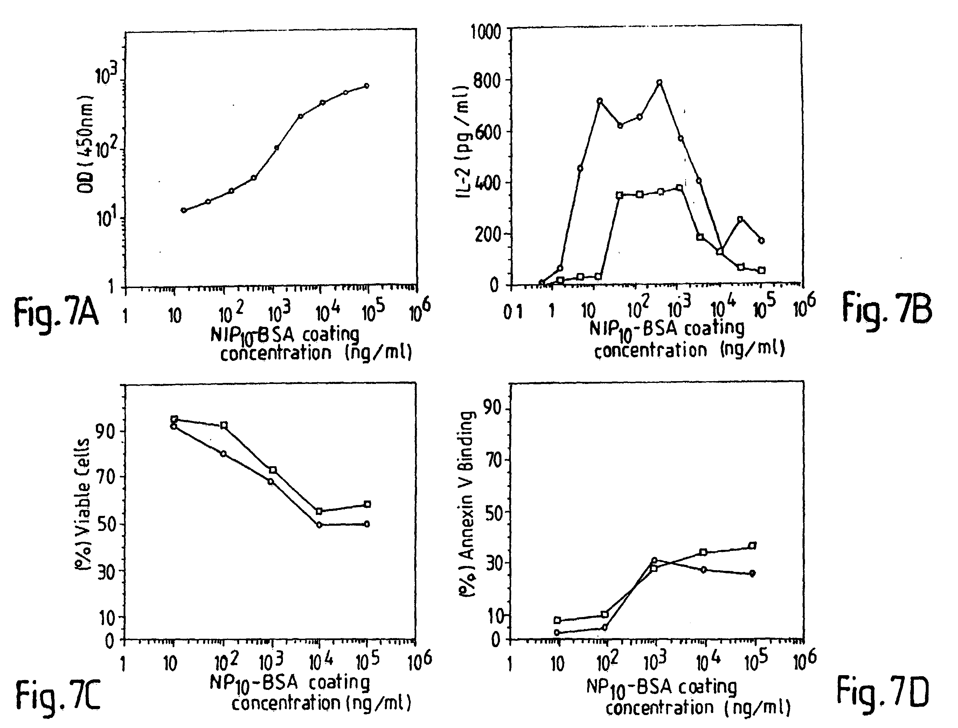

Wells from round-bottom tissue culture treated 96-well plates were passively coated with 100 µl of 10 threefold serial dilutions of NIP₁₀-BSA starting at 100 µg/ml. The relative amount of NIP₁₀-BSA bound to the wells was estimated by ELISA using a soluble B1.8 scFv antibody fragment (15). The binding was detected with a goat antiserum to mouse λ-chain and the peroxidase-conjugated anti-goat/sheep mAb GT-34. The results are shown graphically in Figure 7A.

The inventors then invesitgated the ability of the NIP₁₀-BSA coated plates to stimulate IL-2 production in 1D9 and 2D6 cells. Approximately 5 x 10⁴ cells were stimulated in tetracycline-free medium in round-bottom 96-well plates coated with different amounts of NIP¹⁰-BSA conjugates. Data are from one representative experiment out of three and are shown in Figure 7B. The standard error for IL-2 measurements were less than 15%. In Figure 7B, data for 1D9 cells are shown by open circles, and those for 2D6 cells denoted by filled squares.

Figures 7C and 7D show the results of experiments in which clones 1D9 (open circles) and 2D6 (filled squares) were exposed to different amounts of iNIP10-BSa for 24 hours. In Figure 7C, the number of cells remaining viable is shown (measured cytofluorometrically, as described by Boehme & Lenardo, 1993 Leukemia (Baltimore) 7, 845): the percentage of cell loss was calculated as 100 x [1-(number of viable cells receiving antigenic stimulation/number of viable cells without any treatment)].

Figure 7D shows assessment of apoptosis, measured by Annexin V binding to apoptotic cells, as described by Vermes *et al*, (1995 J. Immunol. Methods 180, 39). In order to distinguish cells that had lost membrane integrity, propidium iodide was added to a final concentration of 10µg/ml before analysis.

The inventors found that when the cells were exposed to a range of different concentrations of iNIP10-BSA (Fig. 7A), optimal IL-2 secretion was seen at low to intermediate antigen density and declined as the antigen density increased beyond this level (Fig. 7B). Suppression of IL-2 secretion at high antigen density was associated with a clear dose-response effect on cell viability (Fig. 7C). Annexin V staining revealed that apoptosis is the mechanism underlying this loss of cell viability, with both clones showing a similar dose-response for apoptosis (Fig. 7D).

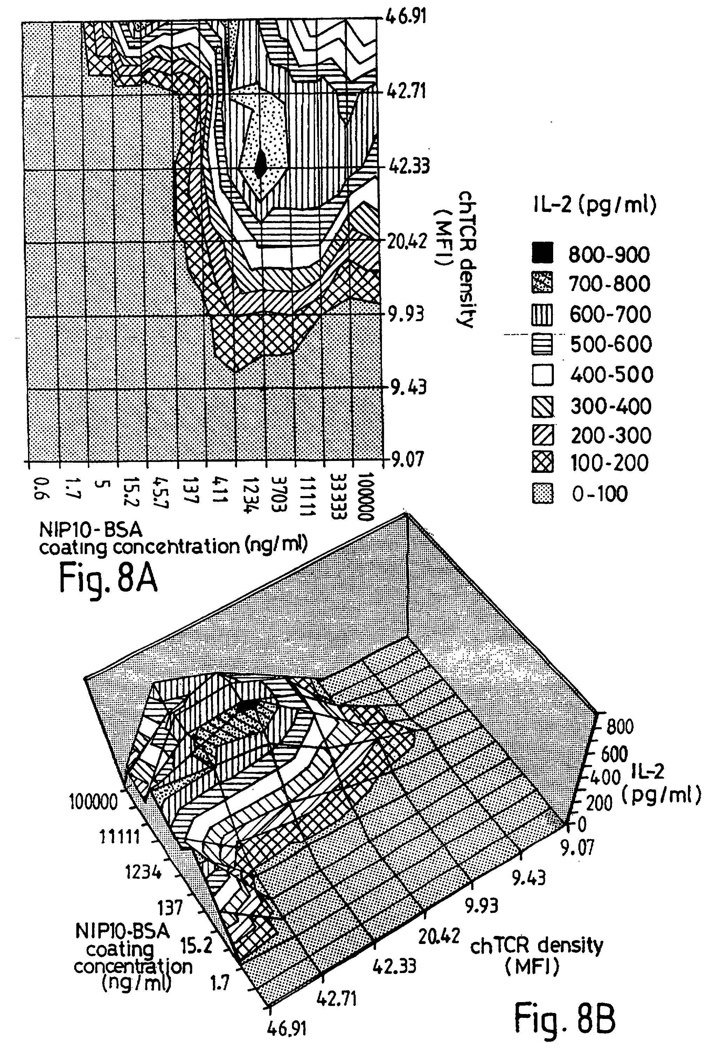

To further explore the relationship between chTCR receptor density, target antigen concentration and T cell response, secretion of IL-2 by 1D9 cells maintained in different concentrations of tetracycline (and therefore expressing different densities of the chTCR) was determined in the presence of varying concentrations of iNIP10-BSA. The data from a representative experiment are shown graphically in Fig. 8 which reveals a numbher of interesting features.

As expected, when the cells express a high density of the chTCR there is a threshold concentration of antigen below which it is not possible to induce maximal IL-2 responses. Likewise, there is a low threshold chTCR receptor density below which it is not possible to induce secretiion of IL-2, even when the cells are exposed to a very high concentration of antigen. Between these two extremes there is an intermediate range of chTCR densities in which the threshold antigen concentration required for stimulation of IL-2 secretion falls progressively as the level of chTCR expression increases. At a given concentration of antigen the magnitude of the IL-2 response increases to a maximum level as the cell surface density of chTCR increases above the threshold level. As receptor density increases further beyond the level that gives maximal IL-2 output there is a progressive damping of the T cell response associated with a loss of T cell viability, presumably a consequence of excessive signal transduction via the chTCR. Receptor-dependent suppression of the IL-2 response was not seen at low antigen concentration, even at the highest level of TCR expression. Figure 8 also reveals that for any given antigen concentration that is capable of optimally stimulating the T cells, there is a corresponding narrow range of receptor densities that correlate withinduction of an optimal T cell response. This 'ideal' receptor density is high at low antigen concentration and diminishes as the antiven concentration increases until it reaches a critical minimum receptor density below which a maximal IL-2 response cannot be elicited, irrespective of antigen concentration. Another interesting feature is the relationship between chTCR density and the suppression of T cell responses at high antigen concentration. The plot reveals that as chTCR density falls, it enters a range in which the T cell response remains optimal over a wide range of high antigen concentrations. Antigen-mediated suppression of the respone is not achieved at these intermediate densities, even at the highest antigen concentration achievable. These data confirm that TCR density sets the threshold concentration of antigen that is required for T cell activation, and provide further evidence that excessive TCR triggering suppresses the T cell response (Critchfield *et al,* 1994 Science **263,** 1139). However, the most significant finding of this study is the revelation that optimal T cell activation is possible only when the TCR density is correctly tuned to respond optimally to the ambient concentration of antigen. The data therefore lend strong support to the hypothesis that T cells undergo receptor density maturation in the course of an immune response.

In addition to their interest and relevance for the study of T cell biology, our observations have implications for the development of therapeutic strategies using adoptive transfer of T cells expressing chimaeric receptors in which single-chain antibody domains are linked to different signalling portions of the TCR/CD3/ζ complex as a means to target the T cells towards cancer antigens (Eshar *et al,* 1993; Brocker *et al*, 1996, both cited above). A major theoretical objection to this approach is that, although expressed more abundantly on cancer cells than on normal tissues, most cancer antigens are not truly tumour specific. However, our data suggest that it should be possible to fix the level of expression of a chimaeric TCR such that an engineered T cell is optimally activated by cancer cells expressing a high density of the target antigen, and not by normal cells which express the same antigen at lower density.

### Example 4

In this example the inventors investigated the ability of T cells, expressing tetracycline-regulatable chimeric TCR molecules, to be stimulated by target (HeLa S3) cells coated with NIP hapten.

Jurkat E6-1 cells were maintained in RPMI 1640 supplemented with 10% FCS, 3mM L-glutamine, antibiotics, 25 mM HEPES buffer (all from GIBCO-BRL, Gaitersburg, MD), and 0.4 mg/ml of hygromycin B (Calbiochem, San Diego, CA as described above), hela S3 cells (ATCC CCL-2.2) were maintained in DMEM supplemented with 10% FCS, 2 mM L-glutamine and antibiotics (GIBCO).

### Hapten Modification of Target Cells

HeLa S3 cells were extensively washed in PBS, counted, and adjusted to a concentration of 10⁷/ml. Different amounts of a fresh 50 mg/ml solution of NIP-CAP-Osu [succinimide ester of 3-nitro-4-hydroxy-5-iodophenylacetic acid spaced with acprioic acid] (Genosys Biotechnologies Inc., Cambridge, UK) in dry dimethylformamide were added to the cell suspension, to give a final concentration range of 1-10µg/ml. The cells were incubated for 1 h at 37°C and then washed three times with 15 ml of cold PBS supplemented with 10% FCS.

### Flow Cytometry

The expression of chTCR molecules was monitored by standard direct immunofluorescence using a FITC-conjugated goat antiserum to mouse λ-chain (Southern Biotechnology Associates, Inc., Birmingham, AL). The relative amount of NIP bound to hapten-modified HeLa S3 cells was estimated by indirect immunofluorescence using saturating amounts of a soluble B1.8 scFv antibody fragment and a FITC-conjugated goat antiserum to mouse λ-chain (Southern Biotechnology Associates). A minimum of 20,000 cells were analyzed with a FACScan (Becton Dickinson).

### T Cell Stimulation

Jurkat 1D9 cells (5 x 10⁵/ml) were preincubated for 48 h in the absence or the presence of tetracycline hydrochloride (Sigma Chemical CO., St Louis, MO), and the surface expression of chTCR was examined by FACS analysis. In each assay 5 x 10⁴ effector Jurkat 1D9 cells expressing different chTCR densities were co-cultured with target HeLa S3 cells expressing different levels of NIP-modified cell-surface proteins at various effector : target (E:T) ratios in medium alone or in the presence of tetracycline at the same concentration as originally used for repression. The cells were incubated in duplicate in round-bottomed 96-well plates (Corning, NY, USA) at 37°C in 5% CO₂ in a final volume of 200µl and after 20 h cell-free supernatants were harvested and assayed for IL-2 activity using an ELISA kit (Genzyme Diagnostic, Cambridge, MA).

### Results

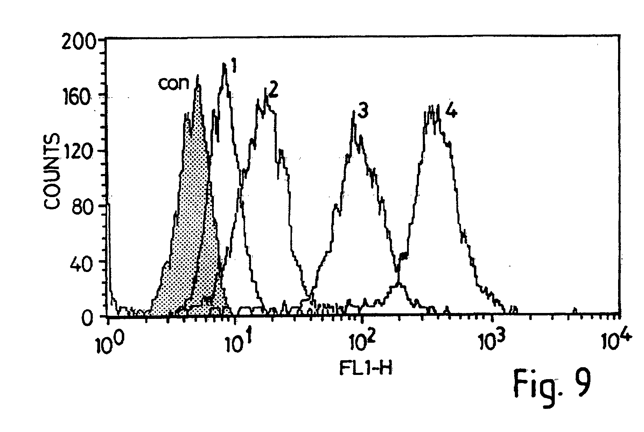

Labelling of Hela S3 cells with different concentrations of NIP-CAP-Osu resulted in the generation of populations of viable cells expressing different levels of hapten-modified cell surface proteins with a homogeneous pattern (Figure 9).

In Figure 9 the GACS profiles of various Hela cell populations are shown. The profile at the extreme left hand end (marked "con") are negative control cells without any NIP-CAP-Osu labelling. Profiles 1-4 are those generated by FACS analysis of Hela S3 cells labelled respectively with 1, 2.5, 5 or 10 µg/ml of the hapten.

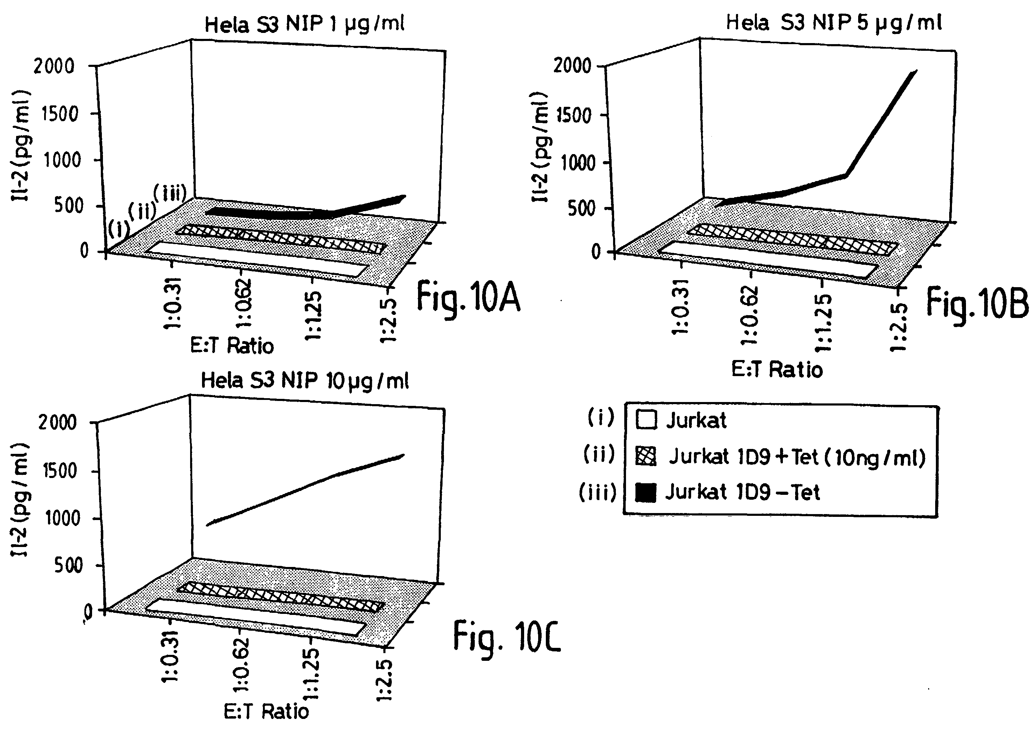

We have previously shown that the chTCR analyzed in this study is able to mediate specific recognition of its cognate antigen (NIP conjugated to BSA), either in solution or immobilised on plastic, resulting in the production of IL-2 by transfected T cells. In this example we found consistently that tetracycline untreated chTCR-expressing 1D9 cells could be specifically triggered, after incubation with NIP-modified stimulator Hela S3 cells, to produce IL-2 in a dose-dependent fashion (see Figures 10A-10C). Interestingly, the level of IL-2 secretion was higher than that observed in the same cell population in response to plastic immobilized NIP-BSA conjugates. This suggests the existence of additional, synergistic or costimulatory signals. Unlabelled Hela S3 cells did not activate Jurkat 1D9 cells (not shown), demonstrating the specificity of the response toward NIP. Nontransduced parental Jurkat cells could not be stimulated to secrete IL-2 by NIP labelled (Figure 10) or unlabelled (not shown) Hela S3 cells. Jurkat 1D9 cells incubated for 24 to 48 h in the presence of 10 ng/ml of tetracycline down-regulated most of the surface chTCR expression (1) and the cells were as a result unresponsive to stimulation with cell-bound hapten, even at high E:T ratios (Figure 10).

### Discussion

The inventors have used a single vector containing all of the components of the TRS for the pharmacological regulation of a foreign gene expressed in a human T cell line. The TRS comprises the tTA gene, usually driven by a constitutive promoter, and a gene of interest immediately downstream of the tTA-responsive promoter (9). To facilitate the application of the TRS to T cells, the inventors constructed a self-contained plasmid vector encoding both components of the TRS, as well as a hygromycin selectable marker gene under the control of a constitutive promoter. The new vector overcomes the efficiency losses inherent in co-transfection with the original two-plasmid based TRS system (9) and ensures the integration of equal copy numbers of the tTA and reporter gene units in a direct cis-configuration at the same chromosome locus.

Using this stable expression system as a model for integrated gene therapy approaches the inventors have demonstrated that a scFv-TCRζ chimeric molecule can be functionally expressed in a human T cell line, and that its expression can be pharmacologically down-modulated leading to loss of responsiveness to the targeted antigen in the genetically modified T cells. In the absence of tetracyclines the level of expression of the chTCR was comparable to that observed when the chimeric gene was driven by the strong CMV IE enhancer/promoter. Efficient tetracycline-dependent suppression of gene expression was observed in all the studied T cell transfectants. Depending on the dose and analogue employed, the expression of chTCRs could be repressed to a greater or lesser extent. This indicates that the TRS is applicable to T cells, achieving functional levels of transactivator expression without any evident toxic squelching effects of the VP16 domain.

Variable potencies have been demonstrated for different tetracycline analogues in previous studies on the TRS with doxycycline exhibiting 100-fold greater potency than tetracycline (18). In our system maximum repression occurred at a concentration of 10 ng/ml tetracycline whereas doxycycline caused full repression of the TRP over the range of 100 pg/ml to 1 ng/ml.

The time-course for the decline in chTCR expression upon exposure to Tet or Dox was short and suggests that, like wild-type TCRζ chains the chimeric TCRζ chains exhibit rapid turnover (19). In this regard it is interesting to note that the turnover of ζ chains in Jurkat cells is similar to that in primary T cells (19). In contrast to the rapid suppression of gene expression, its recovery after removal of doxycycline was much slower. The delay before the commencement of recovery of chTCR gene expression varied in direct proportion to the concentration of doxycycline that was used for repression. This sustained repression and relatively slow recovery of gene expression which occurs upon removal of tetracycline analogues has been observed in other cell types and could be of interest in the development of new T cell immunotherapy approaches and new immune evasion strategies.

Expression of the chimeric TCR conferred responsiveness to NIP-BSA conjugates, as evidenced by IL-2 secretion upon exposure to the antigen. Doxycycline was shown to completely abrogate this antigen responsiveness at concentrations that had no other immunomodulating effects on human T cells, and well below the tissue concentrations that are achieved clinically when doxycycline is used to treat infection (20). Thus, these results show that the use of TRS vectors can provide the means to render reinfused gene-modified T cells unresponsive to their targeted antigen if they cause autoimmune disorders. Likewise, by treating engineered T cells with doxycycline before their administration, it should be possible to switch off expression of the transgene for a predetermined period of time, thereby limiting collateral damage to normal tissues that may express low levels of the targeted antigen.

Interestingly, some residual expression of the chTCR in Tet or Dox treated Jurkat T cells was always detectable by FACS analysis, even when suppression was sufficient to completely abrogate responsiveness to the targeted antigen. This indicates that the number of chTCR molecules expressed in the "off" state is not enough to achieve efficient T cell activation (21), even with the experimental system used,. where the cells were exposed to high concentrations of multivalent antigen. The ability easily to regulate the expression of TCR molecules at defined levels could be relevant to study the stoichiometry of the T cell activation process. On a therapeutic level, this points to the possibility of rendering T cells differentially sensitive to different surface densities of the antigen on target cell membranes.

Complete suppression of transgene expression was not fully achieved in the current study. The characteristics of the employed vector and/or cell type-specific factors may account for the level of transcription observed in the uninduced state (22, 23). In addition, the potential immunogenicity of the tTA protein must be taken into consideration. In the vector that we have employed the transactivator is driven by a constitutive promoter ensuring consistent expression independently of the presence of tetracycline, and may therefore possibly elicit an immune response against the genetically modified T cells, even in the repressed state. However, this is not the case for a newer generation of enhancerless tetracycline-responsive vectors, in which tetracycline prevents the tTA protein from binding to the TRP, thereby suppressing its own expression as well as the expression of the reporter gene by way of an autoregulatory circuit (18, 24). This arrangement gives enhanced suppression of transgene expression (to negligible levels in preliminary experiments and ensures that the abundance of the tTA protein is greatly reduced in the fully repressed state (24).

In summary we have demonstrated the use of a single tetracycline-responsive vector to achieve tetracycline-suppressible expression of a chimeric TCR gene in Jurkat T cells and we have furthermore shown that this provides a convenient method for the pharmacological regulation of the responsiveness of the engineered T cells to their targeted antigen.

### References

1. Mule *et al*, 1984 Science 225:1487.
2. Rosenberg *et al*, 1986 Science 233:1318.
3. Eshhar *et al*, 1993 Proc. Natl. Acad. Sci. USA. 90: 720.
4. Hwu *et al*, 1993 J. Exp. Med. 178: 361.
5. Stancovski *et al* 1993 J. Immunol. 151: 6577.
6. Brocker *et al* 1996 Eur. J. Immunol. 26:1770.
7. Pardoll 1994 Cancer. Curr. Opin. Immunol. 6:705.
8. Yarranton 1992 Curr. Opin. Biotechnol. 3:506.
9. Gossen & Bujard 1992 Proc. Natl. Acad. Sci. USA. 89:5547.
10. Gossen *et al* 1993 Trends Biol. Sci. 18:471.
11. Spits *et al* 1985 Eur. J. Immunol. 15:88.
12. Alvarez-Vallina & Hawkins 1996 Eur. J. Immunol. 26: 2304.
13. Patten *et al* 1992 J. Immunol. 150:2281.
14. Alvarez-Vallina *et al* 1993 J. Immunol.150: 8.
15. Hawkins *et al* 1992 J. Mol. Biol. 226: 889.
16. Weissman *et al* 1988 Proc. Natl. Acad. Sci. USA. 85: 9709.
17. Kloppenburg *et al* 1995 Clin. Exp. Immunol. 102:635-641.
18. Hofmann *et al* 1996 Proc. Natl. Acad. Sci. USA. 93: 5185-5190.
19. Ono *et al* 1995 Immunity. 2:639-644.
20. Houin *et al* 1983 Br. J. Clin. Pharmac. 16:245.
21. Valitutti *et al* 1995 Nature. 375:148.
22. Shockett & Schatz. 1996 Proc. Natl. Acad. Sci. USA. 93:5173.
23. Ackland-Berlund & Leib 1995 BioTechniques. 18:196.
24. Shockett *et al* 1995 Proc. Natl. Acad. Sci. USA. 92:6522.

## Claims

1. A leukocyte transformed with a nucleic acid sequence which expresses a leukocyte-activating molecule in a manner sensitive to the concentration of an exogenous agent, the leukocyte being activated by an interaction between the leukocyte-activating molecule and a leukocyte-stimulating molecule present on the surface of a cell, wherein the leukocyte is differentially reactive to different densities of leukocyte-stimulating molecules and distinguishes between target cells with relatively high densities of leukocyte-stimulating molecules and non-target cells with relatively low densities of leukocyte-stimulating molecules.

2. A B or T lymphocyte according to claim 1.

3. A leukocyte according to claim 1 or 2, wherein the nucleic acid sequence is operably linked to a drug regulatable promoter.

4. A leukocyte according to any one of claims 1, 2 or 3, wherein the leukocyte-stimulating molecule is a tumour-associated antigen, which is expressed at a higher density on a tumour target cell than on non-tumour non-target cells.

5. A leukocyte according to any one of the preceding claims, wherein the leukocyte-stimulating molecule is carcino-embryonic antigen.

6. A leukocyte according to any one of the preceding claims, wherein the leukocyte is a T lymphocyte.

7. A leukocyte according to any one of the preceding claims, wherein the leukocyte-activating molecule is a chimeric polypeptide.

8. A leukocyte according to any one of the preceding claims, wherein the leukocyte-activating molecule comprises an intracellular signalling domain, a transmembrane domain which holds the molecule in the leukocyte cell membrane, and an extracellular domain.

9. A leukocyte according to any one of the preceding claims, wherein the leukocyte-activating molecule comprises the intracellular signalling domain of at least one of the chains of the CD3 complex, or the intracellular signalling domain of a co-stimulatory molecule.

10. A leukocyte according to any one of the preceding claims, wherein the leukocyte-activating molecule comprises a domain which has binding specificity for the leukocyte-stimulating molecule.

11. A leukocyte according to any one of the preceding claims, wherein the exogenous agent is tetracycline or an analogue thereof (as herein defined).

12. A composition for use in a therapeutic method, the composition comprising a plurality of leukocytes according to any one of claims 1-11 in a physiologically acceptable diluent.

13. A method of making a composition according to claim 12, comprising: transforming the leukocytes obtained from a sample from a subject to be treated with a nucleic acid sequence which directs the expression of a leukocyte-activating molecule in a manner sensitive to the concentration of an exogenous agent; and mixing the transformed leukocytes with a physiologically acceptable diluent.

14. Use of a leukocyte according to claim 1, in the preparation of a composition for the elimination of unwanted cells in a human subject.

## Patentansprüche

1. Leukozyt, transformiert mit einer Nucleinsäure-Sequenz, die ein Leukozyt-aktivierendes Molekül auf eine Art exprimiert, die in Bezug auf die Konzentration eines exogenen Mittels sensitiv ist, wobei der Leukozyt durch Interaktion zwischen dem Leukozyt-aktivierenden Molekül und einem Leukozyt-stimulierenden Molekül aktiviert wird, das auf der Oberfläche einer Zelle vorliegt, wobei der Leukozyt in Bezug auf verschiedene Dichten dieser Leukozyt-stimulierenden Moleküle differentiell reaktiv ist und zwischen Zielzellen, die relativ hohe Dichten der Leukozyt-stimulierenden Moleküle aufweisen, und Nicht-Zielzellen, die relativ niedrige Dichten der Leukozyt-stimulierenden Moleküle aufweisen, unterscheidet.

2. B- oder T-Lymphozyt gemäß Anspruch 1.

3. Leukozyt gemäß Anspruch 1 oder 2, wobei die Nucleinsäure-Sequenz in operabler Weise mit einem Pharmazeutikaregulierbaren Promotor verbunden ist.

4. Leukozyt gemäß mindestens einem der Ansprüche 1, 2 oder 3, wobei das Leukozyt-stimulierende Molekül ein Tumorassoziiertes Antigen ist, das auf einer Tumor-Zielzelle mit höheren Dichten exprimiert wird als auf Nicht-Tumor-Nicht-Zielzellen.

5. Leukozyt gemäß mindestens einem der vorhergehenden Ansprüche, wobei das Leukozyt-stimulierende Molekül ein Karzino-embrionisches Antigen ist.

6. Leukozyt gemäß mindestens einem der vorhergehenden Ansprüche, wobei der Leukozyt ein T-Lymphozyt ist.

7. Leukozyt gemäß mindestens einem der vorhergehenden Ansprüche, wobei das Leukozyt-aktivierende Molekül ein chimäres Polypeptid ist.

8. Leukozyt gemäß mindestens einem der vorhergehenden Ansprüche, wobei das Leukozyt-aktivierende Molekül eine intrazelluläre Signaldomäne, eine Transmembrandomäne, die das Molekül in der Membran der Leukozytenzelle hält, und eine extrazelluläre Domäne umfasst.

9. Leukozyt gemäß mindestens einem der vorhergehenden Ansprüche, wobei das Leukozyt-aktivierende Molekül die interzelluläre Signaldomäne von mindestens eine der Ketten des CD3-Komplexes umfasst, oder die intrazelluläre Signaldomäne eines co-stimulierenden Moleküls.

10. Leukozyt gemäß mindestens einem der vorhergehenden Ansprüche, wobei das Leukozyt-aktivierende Molekül eine Domäne umfasst, die eine Bindungsspezifizität für das Leukozyt-stimulierende Molekül aufweist.

11. Leukozyt gemäß mindestens einem der vorhergehenden Ansprüche, wobei das exogene Mittel Tetracyclin oder ein Analog davon (wie hierin definiert) ist.

12. Zusammensetzung zur Verwendung in einem therapeutischen Verfahren, wobei die Zusammensetzung eine Mehrzahl von Leukozyten gemäß mindestens einem der Ansprüche 1 bis 11 in einem physiologisch akzeptablen Verdünner enthält.

13. Verfahren zur Herstellung einer Zusammensetzung gemäß Anspruch 12, umfassend: Das Transformieren der Leukozyten, die aus einer Probe von einem zu behandelnden Individuum erhalten wurden, mit einer Nucleinsäure-Sequenz, die die Expression eines Leukozyt-aktivierenden Moleküls in einer Art steuern, die in Bezug auf die Konzentration eines exogenen Mittels sensitiv ist; und Mischen der transformierten Leukozyten mit einem physiologisch akzeptablen Verdünner.

14. Verwendung eines Leukozyten gemäß Anspruch 1 zur Herstellung einer Zusammensetzung zur Eliminierung von unerwünschten Zellen in einem menschlichen Individuum.

## Revendications

1. Leucocyte transformé par une séquence d'acide nucléique qui exprime une molécule d'activation du leucocyte d'une manière sensible à la concentration en un agent exogène, le leucocyte étant activé par une interaction entre la molécule d'activation du leucocyte et une molécule de stimulation du leucocyte présente à la surface d'une cellule, le leucocyte étant réactif de manière différentielle à différentes densités de molécules d'activation du leucocyte et distinguant entre des cellules cibles ayant des densités élevées en molécules d'activation du leucocyte et des cellules non cibles ayant des densités relativement basses en molécules d'activation du leucocyte.

2. Lymphocyte B ou T selon la revendication 1.

3. Leucocyte selon la revendication 1 ou 2, dans lequel la séquence d'acide nucléique est liée de manière opérationnelle à un promoteur pouvant être régulé par un médicament.

4. Leucocyte selon l'une des revendications 1, 2 ou 3, dans lequel la molécule de stimulation du leucocyte est un antigène associé à une tumeur, qui est exprimé avec une densité plus élevée sur une cellule tumorale cible que sur des cellules non-tumorales non-cibles.

5. Leucocyte selon l'une quelconque des revendications précédentes, dans lequel la molécule de stimulation du leucocyte est un antigène carcino-embryonique.

6. Leucocyte selon l'une quelconque des revendications précédentes, le leucocyte étant un lymphocyte T.

7. Leucocyte selon l'une quelconque des revendications précédentes, dans lequel la molécule d'activation du leucocyte est un polypeptide chimère.

8. Leucocyte selon l'une quelconque des revendications précédentes, dans lequel la molécule d'activation du leucocyte comprend un domaine de signalisation intracellulaire, un domaine transmembranaire qui maintient la molécule dans la membrane cellulaire du leucocyte et un domaine extracellulaire.

9. Leucocyte selon l'une quelconque des revendications précédentes, dans lequel la molécule d'activation du leucocyte comprend le domaine de signalisation intracellulaire d'au moins une des chaînes du complexe CD3, ou le domaine de signalisation intracellulaire d'une molécule de co-stimulation.

10. Leucocyte selon l'une quelconque des revendications précédentes, dans lequel la molécule d'activation du leucocyte comprend un domaine qui possède une spécificité de liaison pour la molécule de stimulation du leucocyte.

11. Leucocyte selon l'une quelconque des revendications précédentes, dans lequel l'agent exogène est la tétracycline ou un analogue de celle-ci (tel que défini dans la présente).

12. Composition pour utilisation dans une méthode thérapeutique, la composition comprenant une pluralité de leucocytes selon l'une des revendications 1 à 11 dans un diluant physiologiquement acceptable.

13. Méthode de préparation d'une composition selon la revendication 12, comprenant la transformation des leucocytes obtenus à partir d'un échantillon d'un sujet à traiter avec une séquence d'acide nucléique qui dirige l'expression d'une molécule d'activation du leucocyte d'une manière sensible à la concentration en un agent exogène ; et le mélange des leucocytes transformés avec un diluant physiologiquement acceptable.

14. Utilisation d'un leucocyte selon la revendication 1, pour la préparation d'une composition pour l'élimination de cellules indésirables chez un sujet humain.
